(19) Europäisches Patentamt
European Patent Office
Office européen des brevets

(11) **EP 4 102 508 A2**

(12) **EUROPEAN PATENT APPLICATION**

(43) Date of publication:
**14.12.2022 Bulletin 2022/50**

(21) Application number: **22177824.4**

(22) Date of filing: **08.06.2022**

(51) International Patent Classification (IPC):
**G16C 10/00** (2019.01)    **G16C 60/00** (2019.01)

(52) Cooperative Patent Classification (CPC):
**G16C 10/00; G16C 60/00;** G16C 20/30

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.06.2021 IN 202121025582**

(71) Applicant: **Tata Consultancy Services Limited Maharashtra (IN)**

(72) Inventors:
• **MAITI, SOUMYADIPTA**
  **411013 Pune, Maharashtra (IN)**
• **BAJPAI, VARNITA**
  **411013 Pune, Maharashtra (IN)**
• **MISHRA, SHASHANK**
  **411013 Pune, Maharashtra (IN)**
• **RAI, BEENA**
  **411013 Pune, Maharashtra (IN)**

(74) Representative: **Goddar, Heinz J.**
  **Boehmert & Boehmert**
  **Anwaltspartnerschaft mbB**
  **Pettenkoferstrasse 22**
  **80336 München (DE)**

(54) **GENERATING MOLECULAR DYNAMICS POTENTIALS AND SIMULATING THEREOF FOR PREDICTING PROPERTIES OF MULTI-ELEMENT ALLOY STRUCTURES**

(57) Traditionally, new alloy development and processing involved various high-end expansive experiments, huge development time and cost of required man-hours. One of the major issues, which limits the ability for materials scientists to design metallic materials from atoms using Molecular Dynamics (MD), is the lack of accurate interatomic molecular dynamics potentials (MDPs). Suitable MDPs of desired alloy systems enable new alloy compositions and related properties, but however, this is very difficult and time-consuming process. The present disclosure enables developing molecular dynamics potential for new/traditional metallic alloys for their simulated structural, thermodynamic, and mechanical property predictions. Present disclosure provides systems and methods for generating MDP for multi-element alloy systems wherein both Body Centered Cubic (BCC) element type and/or a Face Centered Cubic (FCC) element type are combined. Pure elements and multi-element alloys of combinations of BCC and FCC elements are modeled for predicting their various structural, thermodynamic, and mechanical properties.

```
obtaining one or more input physical parameters
corresponding to a multi-element alloy structure          — 202

classifying the one or more input physical parameters as
one of a first element type, or a second element type to
obtain at least one of a first set of elements and a second — 204
set of elements respectively

computing one or more of (i) an embedding energy function,
and (ii) an atomic electron density for each input physical
parameter of the at least one of the first set of elements and — 206
the second set of elements

scaling, by using a scaling factor, the one or more of (i) the
embedding energy function, and (ii) the electron density,
computed for each input physical parameter of the at least
one of the first set of elements and the second set of         — 208
elements, to obtain a set of scaled parameters and an
elemental interaction pair potential function

identifying one or more dissimilar-type pair interaction
potential parameters based on the set of scaled parameters — 210
and the elemental interaction pair potential function

sequencing the one or more identified dissimilar-type pair
interaction potential parameters and one or more similar-
type pair interaction potential parameters to obtain a        — 212
sequence of the pair type interaction potential parameters

generating a molecular dynamics potential (MDP) file based
on the sequence of the one or more type pair interaction     — 214
potential parameters
```

**FIG. 2**

**Description**

CROSS-REFERENCE TO RELATED APPLICATIONS AND PRIORITY

The present application claims priority to Indian application no. 202121025582, filed on June 9, 2021.

TECHNICAL FIELD

The disclosure herein generally relates to simulation techniques for multi-element alloy structures, and, more particularly, to generating molecular dynamics potentials and simulating thereof for predicting properties of multi-element alloy structures.

BACKGROUND

Alloys are widely used because of its superiority in terms of strength, toughness, thermal and other mechanical properties than its constituent elements. It is difficult to determine structural, thermodynamic, and mechanical properties of new alloys precisely because alloys do not just follow predictable rule of mixture of constituting element properties. As a result, much costly and time-consuming experimental testing are required in the development and investigating properties of new metallic alloys.

Classical molecular dynamics (MD) simulations have become ubiquitous tool for simulating various nanoscopic to macroscopic properties in many complex alloy systems such as local relaxed structure, thermodynamic changes, short-range ordering, dislocation movement and strengthening effect. One of the major issues, which limits the ability for materials scientists to design, develop and process metallic materials from their atomic structure using classical MD, is the lack of accurate and suitable interatomic molecular dynamics potentials (MDPs) for the targeted alloy system. Interatomic potentials describe interactions between atoms of alloy systems using computationally efficient functions to solve the atomic movements. These potentials can range from simple pair interactions to more complex formulations that involve the local atomic electron density, bonds, angles, and torsion. Of these, the embedded-atom method (EAM) has been used widely because it can accurately model thermodynamic properties, stability of the structure and defects in metals. Although many good-quality EAM potentials exists for a considerable number of elemental metals, suitable potentials for alloy systems are sparsely available in literature. This makes fast computational exploration of new alloy compositions difficult.

SUMMARY

Embodiments of the present disclosure present technological improvements as solutions to one or more of the above-mentioned technical problems recognized by the inventors in conventional systems. For example, in one aspect, there is provided a processor implemented method for generating molecular dynamics potentials and simulating thereof for predicting properties of multi-element alloy structures. The method comprises obtaining, via one or more hardware processors, one or more input physical parameters corresponding to a multi-element alloy structure; classifying, via the one or more hardware processors, the one or more input physical parameters as one of a first element type, or a second element type to obtain at least one of a first set of elements and a second set of elements respectively; computing, via the one or more hardware processors, one or more of (i) an embedding energy function, and (ii) an atomic electron density for each input physical parameter of the at least one of the first set of elements and the second set of elements; scaling, by using a scaling factor, via the one or more hardware processors, the one or more of (i) the embedding energy function, and (ii) the atomic electron density, computed for each input physical parameter of the at least one of the first set of elements and the second set of elements, to obtain a set of scaled parameters and an elemental interaction pair potential function; identifying, via the one or more hardware processors, one or more dissimilar-type pair interaction potential parameters based on the set of scaled parameters and the elemental interaction pair potential function; sequencing, via the one or more hardware processors, the one or more identified dissimilar-type pair interaction potential parameters and one or more similar-type pair interaction potential parameters to obtain a sequence of the pair type interaction potential parameters; and generating, via the one or more hardware processors, a molecular dynamics potential (MDP) file based on the sequence of the one or more type pair interaction potential parameters. In an embodiment, the order of the sequence of pair type interaction potential parameters is determined based on a sequence of one or more element types comprised in the one or more input physical parameters.

In an embodiment, the method further comprises simulating the MDP file to predict at least one of (i) one or more structural properties, (ii) one or more thermodynamic properties, and (iii) one or more mechanical properties of the multi-element alloy structure.

In an embodiment, the first set of elements and the second set of elements are distinct from each other.

In another embodiment, the first element type and the second element type are one of a body centered cubic (BCC) element type or a face centered cubic (FCC) element type.

In an embodiment, the scaling factor is calculated based on a regression analysis of the atomic electron density at embedding energy function minima and equilibrium electron density values.

In another aspect, there is provided a system for generating molecular dynamics potentials and simulating thereof for predicting properties of multi-element alloy structures. The system comprises: a memory storing instructions; one or more communication interfaces; and one or more hardware processors coupled to the memory via the one or more communication interfaces, wherein the one or more hardware processors are configured by the instructions to: obtain one or more input physical parameters corresponding to a multi-element alloy structure; classify the one or more input physical parameters as one of a first element type, or a second element type to obtain at least one of a first set of elements and a second set of elements respectively; compute one or more of (i) an embedding energy function, and (ii) an atomic electron density for each input physical parameter of the at least one of the first set of elements and the second set of elements; scale, by using a scaling factor, the one or more of (i) the embedding energy function, and (ii) the atomic electron density, computed for each input physical parameter of the at least one of the first set of elements and the second set of elements, to obtain a set of scaled parameters and an elemental interaction pair potential function; identify one or more dissimilar-type pair interaction potential parameters based on the set of scaled parameters and the elemental interaction pair potential function; sequence the one or more identified dissimilar-type pair interaction potential parameters and one or more similar-type pair interaction potential parameters to obtain a sequence of the pair type interaction potential parameters; and generate a molecular dynamics potential (MDP) file based on the sequence of the one or more type pair interaction potential parameters.

In an embodiment, the one or more hardware processors are further configured by the instructions to simulate the MDP file to predict at least one of (i) one or more structural properties, (ii) one or more thermodynamic properties, and (iii) one or more mechanical properties of the multi-element alloy structure.

In an embodiment, the first set of elements and the second set of elements are distinct from each other.

In another embodiment, the first element type and the second element type are one of a body centered cubic (BCC) element type or a face centered cubic (FCC) element type.

In an embodiment, the scaling factor is calculated based on a regression analysis of the atomic electron density at embedding energy function minima and equilibrium electron density values.

In yet another aspect, there are provided one or more non-transitory machine-readable information storage mediums comprising one or more instructions which when executed by one or more hardware processors cause a method for generating molecular dynamics potentials and simulating thereof for predicting properties of multi-element alloy structures. The method comprises obtaining, via one or more hardware processors, one or more input physical parameters corresponding to a multi-element alloy structure; classifying, via the one or more hardware processors, the one or more input physical parameters as one of a first element type, or a second element type to obtain at least one of a first set of elements and a second set of elements respectively; computing, via the one or more hardware processors, one or more of (i) an embedding energy function, and (ii) an atomic electron density for each input physical parameter of the at least one of the first set of elements and the second set of elements; scaling, by using a scaling factor, via the one or more hardware processors, the one or more of (i) the embedding energy function, and (ii) the atomic electron density, computed for each input physical parameter of the at least one of the first set of elements and the second set of elements, to obtain a set of scaled parameters, and an elemental interaction pair potential function; identifying, via the one or more hardware processors, one or more dissimilar-type pair interaction potential parameters based on the set of scaled parameters and the elemental interaction pair potential function; sequencing, via the one or more hardware processors, the one or more identified dissimilar-type pair interaction potential parameters and one or more similar-type pair interaction potential parameters to obtain a sequence of the pair type interaction potential parameters; and generating, via the one or more hardware processors, a molecular dynamics potential (MDP) file based on the sequence of the one or more type pair interaction potential parameters. In an embodiment, the order of the sequence of pair type interaction potential parameters is determined based on a sequence of one or more element types comprised in the one or more input physical parameters.

In an embodiment, the method further comprises simulating the MDP file to predict at least one of (i) one or more structural properties, (ii) one or more thermodynamic properties, and (iii) one or more mechanical properties of the multi-element alloy structure.

In an embodiment, the first set of elements and the second set of elements are distinct from each other.

In another embodiment, the first element type and the second element type are one of a body centered cubic (BCC) element type or a face centered cubic (FCC) element type.

In an embodiment, the scaling factor is calculated based on a regression analysis of the atomic electron density at embedding energy function minima and equilibrium electron density values.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for generating molecular dynamics potentials and simulating thereof for predicting properties of multi-element alloy structures, in accordance with an embodiment of the present disclosure.

FIG. 2 illustrates an exemplary flow diagram of a method for generating molecular dynamics potentials and simulating thereof for predicting properties of multi-element alloy structures using the system of FIG. 1 in accordance with an embodiment of the present disclosure.

FIG. 3 depicts a graphical representation of a comparison and scaling of atomic electron density values at the Embedding Energy minima for a body centered cubic (BCC) element type and/or a face centered cubic (FCC) element type molecular dynamics potential (MDP) framework, using the system of FIG. 1, in accordance with an embodiment of the present disclosure.

FIG. 4 depicts a graphical representation of a comparison and scaling of atomic electron density values at the next neighbor atomic distance for the body centered cubic (BCC) element type and/or the face centered cubic (FCC) element type MDP framework, using the system of FIG. 1, in accordance with an embodiment of the present disclosure.

FIG. 5 depicts a graphical representation of an elemental interaction pair potential function, in accordance with an embodiment of the present disclosure.

FIG. 6 depicts a graphical representation illustrating a comparison of Embedding energy minima at equilibrium atomic electron density $(\rho_e)$ of elements calculated by BCC and FCC MDP framework, using the system of FIG. 1, in accordance with an embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Metallic alloys are known for their superior properties in terms of enhanced strength and toughness at ambient and elevated temperatures which is applicable for many structural and applications. Traditionally, the new alloy development and alloy processing involved various high-end expansive experiments, development time for 10-20 years and cost of required man-hours. Nowadays with the rise in computation power, molecular dynamics (MD) simulation has become a major computational tool, which simulates metallic alloy systems for their nanostructure, thermodynamic and mechanical properties by involving molecular dynamics potentials (MDP). This MD based simulations can reduce the number of required experiments and subsequently significantly reduce the cost of alloy development and speed up the development works. However, one of the major issues, which limits the ability for materials scientists to design metallic materials from the atoms using classical Molecular Dynamics, is the lack of accurate interatomic molecular dynamics potentials (MDPs). Suitable MDPs of the desired alloy systems makes fast exploration of new alloy compositions and related properties, which is very difficult and time-consuming process otherwise through experimental routes. The present disclosure provides systems and methods for developing the essential molecular dynamics potential for new or traditional metallic alloys for their computer simulated structural, thermodynamic, and mechanical property predictions. More specifically, embodiments of the present disclosure provide systems and methods for generating MDP for multi-element alloy systems wherein both Body Centered Cubic (BCC) element type and/or a Face Centered Cubic (FCC) element type are combined. Pure elements and multi-element alloys of combinations of BCC and FCC elements are modeled for predicting their various structural, thermodynamic, and mechanical properties.

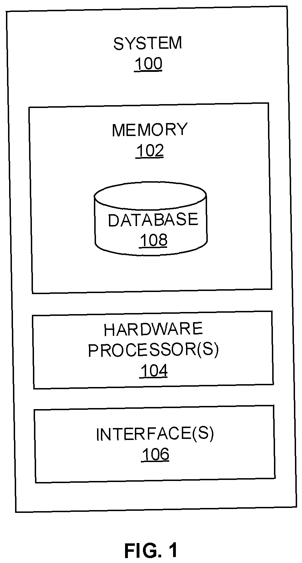

Referring now to the drawings, and more particularly to FIG. 1 through 6, where similar reference characters denote corresponding features consistently throughout the figures, there are shown preferred embodiments and these embodiments are described in the context of the following exemplary system and/or method.

FIG. 1 illustrates an exemplary block diagram of a system for generating molecular dynamics potentials and simulating thereof for predicting properties of multi-element alloy structures, in accordance with an embodiment of the present disclosure. In an embodiment, the system 100 includes one or more processors 104, communication interface device(s) or input/output (I/O) interface(s) 106, and one or more data storage devices or memory 102 operatively coupled to the one or more processors 104. The one or more processors 104 may be one or more software processing modules and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the device 100 can be implemented in a variety of computing systems, such as laptop computers, notebooks, hand-held devices, workstations, mainframe computers, servers, a network cloud and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment a database 108 can be stored in the memory 102, wherein the database 108 may comprise, but are not limited to information pertaining to physical parameters corresponding to a multi-element alloy structure, output(s) generated by one or more simulation technique(s), one or more modeling technique(s), scaling techniques, scaling factors, etc. In an embodiment, the memory 102 may store the one or more modeling technique(s), the one or more simulation technique(s), which are execcuted by the one or more hardware processors 104 to perform the methodology described herein. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

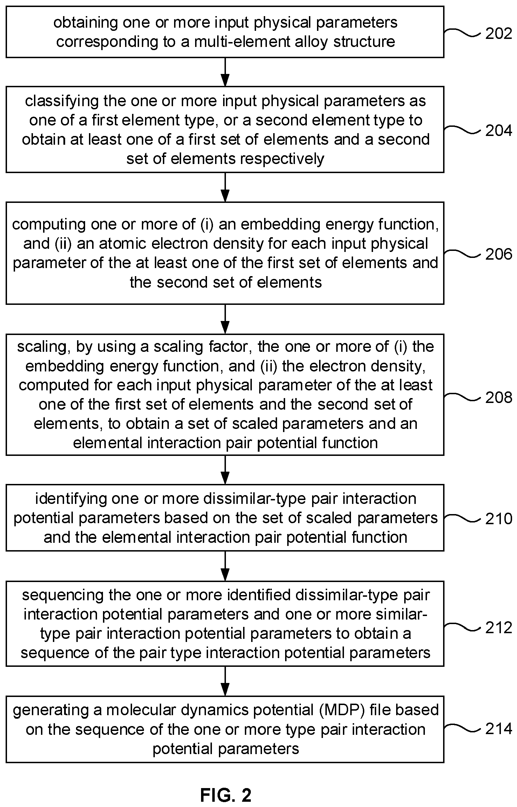

FIG. 2, with reference to FIG. 1, illustrates an exemplary flow diagram of a method for generating molecular dynamics potentials and simulating thereof for predicting properties of multi-element alloy structures using the system 100 of FIG. 1, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to the components of the system 100 as depicted in FIG. 1, and the flow diagram. In an embodiment of the present disclosure, at step 202, the one or more hardware processors 104 obtain one or more input physical parameters corresponding to a multi-element alloy structure. In an embodiment, the one or more input physical parameters comprise lattice parameter, cohesive energy, vacancy formation energy, second order elastic constants, and the like. Table 1 and Table 2 depict the one or more input physical parameters corresponding to the multi-element alloy structure (e.g., say Nickel-Vanadium (NiV or Ni-V) alloy structure, palladium-manganese (PdMn or Pd-Mn) alloy structure.

Table 1

| Metal | $a$ (Å) | $E_c$ (eV) | $E_f$ (eV) | $C_{11}$(eV) | $C_{12}$(eV) | $C_{44}$(eV) |
|-------|---------|-----------|-----------|-------------|-------------|-------------|
| Ta | 3.3025 | 9.71 | 2.95 | 1.648 | 0.986 | 0.516 |
| Mo | 3.147 | 8.691 | 3.1 | 3.017 | 0.954 | 0.786 |
| W | 3.165 | 11.070 | 3.95 | 3.263 | 1.267 | 0.9987 |
| Fe | 2.866 | 5.479 | 1.79 | 1.342 | 0.89 | 0.557 |

Table 2

| Parameters | Fe | Mo | Ta | w | Ni | Pd |
|-----------|--------|--------|--------|--------|--------|--------|
| $r_e$ | 2.481 | 2.728 | 2.860 | 2.740 | 2.488 | 2.750 |
| $f_e$ | 1.885 | 2.723 | 3.086 | 3.487 | 2.007 | 1.595 |
| $p_e$ | 20.041 | 29.354 | 33.787 | 37.234 | 27.562 | 21.335 |
| $p_s$ | 20.041 | 29.354 | 33.787 | 37.234 | 27.930 | 21.940 |
| $\alpha$ | 9.818 | 8.393 | 8.489 | 8.900 | 8.383 | 8.697 |
| $\beta$ | 5.236 | 4.476 | 4.527 | 4.746 | 4.471 | 4.638 |

(continued)

| Parameters | Fe | Mo | Ta | w | Ni | Pd |
|---|---|---|---|---|---|---|
| $A$ | 0.392 | 0.708 | 0.611 | 0.882 | 0.429 | 0.406 |
| $B$ | 0.646 | 1.120 | 1.032 | 1.394 | 0.633 | 0.599 |
| $\kappa$ | 0.170 | 0.137 | 0.176 | 0.139 | 0.443 | 0.397 |
| $\lambda$ | 0.340 | 0.275 | 0.353 | 0.278 | 0.820 | 0.754 |
| $F_{n0}$ | -2.534 | -3.692 | -5.103 | -4.946 | -2.693 | -2.321 |
| $F_{n1}$ | -0.059 | -0.178 | -0.405 | -0.148 | -0.076 | -0.473 |
| $F_{n2}$ | 0.193 | 0.380 | 1.112 | 0.365 | 0.241 | 1.615 |
| $F_{n3}$ | -2.282 | -3.133 | -3.585 | -4.432 | -2.375 | -0.231 |
| $F_0$ | -2.540 | -3.710 | -5.140 | -4.960 | -2.700 | -2.360 |
| $F_1$ | 0.000 | 0.000 | 0.000 | 0.000 | 0.000 | 0.000 |
| $F_2$ | 0.200 | 0.875 | 1.640 | 0.661 | 0.265 | 1.481 |
| $F_3$ | -0.148 | 0.776 | 0.221 | 0.348 | -0.152 | -1.675 |
| $\eta$ | 0.391 | 0.790 | 0.848 | -0.582 | 0.469 | 1.130 |
| $F_e$ | -2.539 | -3.712 | -5.141 | -4.961 | -2.699 | -2.352 |

At step 202 of the present disclosure, the one or more hardware processors 104 classify the one or more input physical parameters as one of a first element type, or a second element type to obtain at least one of a first set of elements and a second set of elements respectively. The first set of elements and the second set of elements are distinct from each other. For example, the first element type and the second element type are one of a body centered cubic (BCC) element type or a face centered cubic (FCC) element type. It is to be understood by a person having ordinary skill in the art or person skilled in the art that input physical parameters pertaining to BCC and FCC elements shall not be construed as limiting the scope of the present disclosure. More specifically, Table 1 depicts the input physical parameters of BCC elements and Table 2 depicts the input physical parameters of FCC elements both of which are obtained from experimental or theoretical data, in one example embodiment of the present disclosure. In the experiments conducted by the present disclosure, two different suitable EAM MDP alloy development methodologies were chosen. The first EAM MDP methodology is only reserved for BCC elements. The second EAM MDP methodology is developed predominantly for FCC elements. But in the second EAM MDP methodology, there is also a provision for developing MDP for some BCC elements.

Referring to steps of FIG. 2, at step 206 of the present disclosure, the one or more hardware processors 104 compute one or more of (i) an embedding energy function, and (ii) an atomic electron density for each input physical parameter of the at least one of the first set of elements and the second set of elements. Input parameters such as lattice constant, cohesive energy, vacancy formation energy and other elastic constants for the models are taken from experimental values or Density Functional Theory (DFT) calculations available in the literature. Using the mathematical formulation of different methodologies for BCC and FCC type elements, MDP component tables of embedding energy, atomic electron density and pairwise interaction potential between dissimilar elements were determined/computed for all different elements. Below description illustrates computation of one or more of (i) an embedding energy function, (ii) an atomic electron density, and (iii) an elemental interaction pair potential function for BCC elements.

An analytical nearest neighbor potential for BCC element-based alloys is developed first for a few BCC elements, in one example embodiment of the present disclosure. The basic governing equations of EAM model as described earlier are:

$$E_{total} = \sum F(\rho_i) + \frac{1}{2} \sum_{i,j(i \neq j)} \Phi(r_{ij})$$

$$\rho_i = \sum_{j \neq i} f(r_{ij})$$

In order to apply the model, the embedding function $F(\rho)$, atomic electron density function $f(r)$, and the two-body pair interaction potential function $\Phi(r)$ must be known. The atomic electron density around each BCC atom is given by:

$$f_r = f_e \, (r_{1e}/r)^\beta$$

where $f_e$ is a factor which is determined by

$$f_e = E_c/\Omega$$

$E_c$ is the cohesive energy, $\Omega$ is the atomic volume, $r_{1e}$ is the equilibrium first neighbor distance, and $\beta$ is an adjustable parameter taken as $\beta=6$ for all of the BCC alloy systems.

$$F(\rho) = -(Ec - E_f)((1 - \ln(\rho/\rho e))^n)(\rho/\rho e)^n$$

Here $E_f$ is the unrelaxed vacancy formation energy, $\rho$ is the equilibrium atomic electron density, and $n$ is a parameter that is given by:

$$n = \frac{1}{\beta}\left[\frac{9\Omega B - 15\Omega G}{Ec - Ef}\right]^{\frac{1}{2}}$$

where $B$ is the bulk modulus and $G$ is the Voigt average shear modulus.

The monoatomic/ single-element pair interaction potential is taken as cubic spline function and is illustrated as below expression:

$$\Phi(r) = k_3\left[\frac{r}{r_{1e}} - 1\right]^3 + k_2\left[\frac{r}{r_{1e}} - 1\right]^2 + k_1\left[\frac{r}{r_{1e}} - 1\right]^1 + k_0$$

where, $k$ constants depend upon a combination of elastic constants and elastic anisotropy.

However, the pair interaction potential of two dissimilar type BCC elements, $a$ and $b$ is given as alloy potential is:

$$\Phi_{ab} = \frac{1}{2}\left[\frac{f^b(r)}{f^a(r)}\Phi_{aa} + \frac{f^a(r)}{f^b(r)}\Phi_{bb}\right]$$

where, $\Phi_{aa}$ and $\Phi_{bb}$ are monoatomic potentials given by monoatomic models. $f^a(r)$ and $f^b(r)$ are atomic electron density function for $a$- and $b$-type atoms.

Below description illustrates computation of one or more of (i) an embedding energy function, (ii) an atomic electron density, and (iii) an elemental interaction pair potential function for FCC elements.

In this EAM alloy potential model, the generalized elemental pair potentials are expressed as:

$$\Phi(r) = \frac{A\exp\left[-\alpha\left(\frac{r}{re} - 1\right)\right]}{1 + \left(\frac{r}{re} - k\right)^{20}} - \frac{B\exp\left[-\beta\left(\frac{r}{re} - 1\right)\right]}{1 + \left(\frac{r}{re} - \lambda\right)^{20}}$$

where $r_e$ is the equilibrium spacing between nearest neighbors, $A, B, \alpha,$ and $\beta$ are four adjustable parameters, and $k$ and $\lambda$ are two additional parameters for the cutoff distance of interaction. The electron density function around each atom is taken as:

$$f(r) = \frac{f_e \, exp\left[-\beta\left(\frac{r}{re} - 1\right)\right]}{1 + \left(\frac{r}{re} - \lambda\right)^{20}}$$

Embedding energy functions that works well over a wide range of atomic electron density require that three equations be used to separately fit three different atomic electron density ranges. For a smooth variation of the embedding energy, these equations are required to match values and slopes at their junctions.

To have embedding energy functions that can work well over a wide range of atomic electron density $\rho$, we have used three equations to separately fit to different atomic electron density ranges: $\rho < \rho_n$, $\rho_n < \rho < \rho_0$ and $\rho_0 < \rho$. By using $\rho_n = 0.85\rho_e$ and $\rho_0 = 1.15\rho_e$ where $\rho_e$ is the equilibrium electron density, it is ensured that all equilibrium properties can be fitted in the atomic electron density range: $\rho_n < \rho < \rho_0$. These equations are given by:

$$F(\rho) = \sum_{i=0}^{3} F_{ni}\left(\frac{\rho}{\rho_n} - 1\right)^i, \rho < \rho_n, \ \rho_n = 0.85\rho_e$$

$$F(\rho) = \sum_{i=0}^{3} F_i\left(\frac{\rho}{\rho_e} - 1\right)^i, \ \rho_n < \rho < \rho_0, \ \rho_0 = 1.15\rho_e$$

$$F(\rho) = F_e\left[1 - \ln\left\{\frac{\rho}{\rho_s}\right\}^n\right]\left\{\frac{\rho}{\rho_s}\right\}^n, \ \rho_0 < \rho$$

The pair potential is defined as similar to BCC model, which includes monoatomic potential and the atomic electron density function of both $a$ and $b$ atom types.

$$\Phi_{ab} = \frac{1}{2}\left[\frac{f^b(r)}{f^a(r)}\Phi_{aa} + \frac{f^a(r)}{f^b(r)}\Phi_{bb}\right]$$

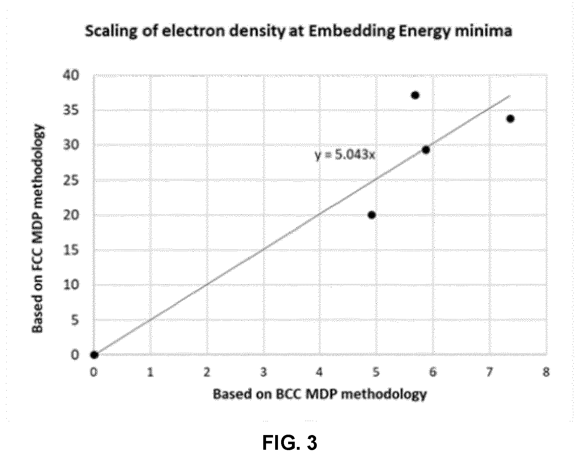

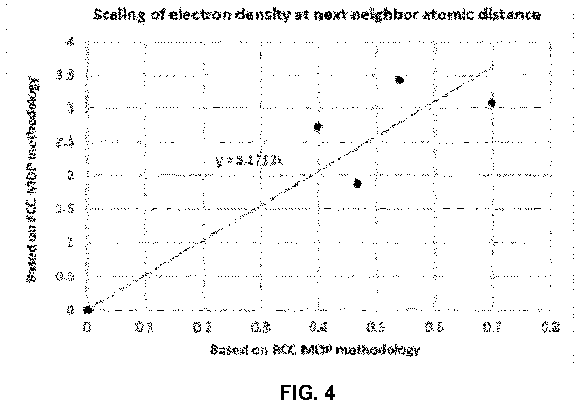

In the FIGS. 3 and 4, the electron densities of elements Fe, Mo, Ta and W are graphically plotted for a scaling factor determination between the 1st MDP methods (BCC method) and 2nd MDP method (FCC method). These values of electron density at embedding energy minima, atomic electron density at next neighbor distance and embedding energy at the minima for both 1st and 2nd MDP methods and are presented in below Table. 3. More specifically, Table. 3 depicts values of atomic electron density at embedding energy minima, electron density at next neighbor distance and embedding energy at minima for different elements and different MDP methods (1st MDP method for BCC and 2nd MDP method for FCC), respectively. The expressions, electron density and atomic electron density may be interchangeably used herein.

Table 3

| Elements | Embedded energy min (1st MDP method) | Embedded energy min (2nd MDP method) | Electron density at Embedded energy min (1st MDP method) | Electron density at Embedded energy min (2nd MDP method) | Next neighbor Electron density (1st MDP method) | Next neighbor Electron density (2nd MDP method) |
|---|---|---|---|---|---|---|
| **Fe** | -3.69 | -2.54 | 4.91 | 20.04 | 0.466 | 1.88 |
| **Mo** | -5.59 | -3.71 | 5.87 | 29.35 | 0.399 | 2.726 |

(continued)

| Elements | Embedded energy min (1st MDP method) | Embedded energy min (2nd MDP method) | Electron density at Embedded energy min (1st MDP method) | Electron density at Embedded energy min (2nd MDP method) | Next neighbor Electron density (1st MDP method) | Next neighbor Electron density (2nd MDP method) |
|---|---|---|---|---|---|---|
| **Ta** | -7.12 | -5.14 | 7.36 | 33.79 | 0.698 | 3.087 |
| **W** | -6.76 | -4.96 | 5.68 | 37.23 | 0.539 | 3.43 |

Referring to steps of FIG. 2, at step 208 of the present disclosure, the one or more hardware processors 104 scale the one or more of (i) the embedding energy function, and (ii) the atomic electron density, computed for each input physical parameter of the at least one of the first set of elements and the second set of elements, to obtain a set of scaled parameters and an elemental interaction pair potential function. In an embodiment of the present disclosure, the step of scaling the one or more of (i) the embedding energy function, and (ii) the atomic electron density for each element of the at least one of the first set of elements and the second set of elements, to obtain a set of scaled parameters, comprises: applying a scaling factor on the one or more of (i) the embedding energy function, and (ii) the atomic electron density for each element of the at least one of the first set of elements and the second set of elements. More specifically, the scaling factor is calculated based on a regression analysis of the atomic electron density at embedding energy function minima and equilibrium electron density values. Below description illustrates the step of scaling the one or more of (i) the embedding energy function, and (ii) the atomic electron density for each element of the at least one of the first set of elements and the second set of elements.

The two different BCC and FCC methods of MDP development methodologies described above need to be merged to produce MDP for mixed BCC-FCC element alloys. MDP table components such as Embedding Function and atomic Electron Density are subjected to numerical scaling. To find the scaling constant a few elements such as Fe, Mo, Ta, W were chosen for which EAM potentials could be made by both BCC and FCC framework procedure routes. Then the Embedding Function $F(\rho)$ and atomic Electron Density $f(r)$ obtained from the FCC procedure is scaled and transformed by:

$$F(\rho) \rightarrow F(\rho/s)$$

$$f(r) \rightarrow f(r)/s$$

where s is the scaling factor, $\rho$ is an electron density, and $r$ is the distance between atoms.

To calculate the scaling factor $s$, atomic electron density at embedding energy function minima and equilibrium electron density of the selected elements is calculated from the BCC and FCC MDP development methods. The linear scaling factor is calculated from the regression analysis of the values of both electron atomic density at embedding energy function minima and equilibrium atomic electron density values calculated by BCC and FCC methods. The average value of the scaling constant is taken from the two graphs of comparison of atomic electron density at embedding function minima plot and equilibrium atomic electron density plot for obtained from both BCC and FCC methods. FIG. 3, with reference to FIGS. 1 and 2, depicts a graphical representation of a comparison and scaling of atomic electron density values at the Embedding Energy minima for body centered cubic (BCC) element type and/or the face centered cubic (FCC) element type MDP framework, using the system 100 of FIG. 1, in accordance with an embodiment of the present disclosure. More specifically, in FIG. 3, the atomic electron density at the Embedding Energy minima is compared between BCC and FCC MDP framework.

FIG. 4, with reference to FIGS. 1 through 3, depicts a graphical representation of a comparison and scaling of atomic electron density values at the next neighbor atomic distance for the body centered cubic (BCC) element type and/or the face centered cubic (FCC) element type MDP framework, using the system 100 of FIG. 1, in accordance with an embodiment of the present disclosure. More specifically, in FIG. 4, the atomic electron density at next neighbor atomic distance is compared between BCC and FCC MDP framework. According to experiments conducted by the present disclosure, regression analysis on FIG. 3 resulted in a scaling factor of 5.043 and regression analysis on FIG. 4 resulted in a scaling factor of the scaling factor is 5.1712 (scaling factor is depicted beside the regression lines in the respective graphical representations). Since the average of these scaling factor from FIG. 3 and 4 is close to 5, this value was fixed for all subsequent MDP development for combined BCC and FCC elements by the present disclosure.

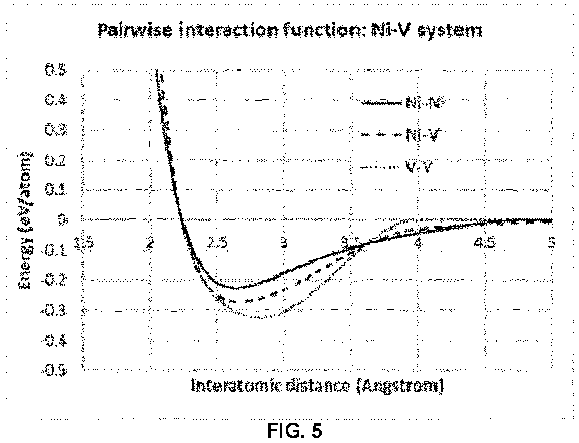

In the present disclosure, after the scaling analysis of atomic electron density and Embedding functions has been completed, the Pairwise-interaction potentials between BCC and FCC type elements can be constructed. FIG. 5, with reference to FIGS. 1 through 4, depict a graphical representation of an elemental interaction pair potential function, in accordance with an embodiment of the present disclosure. More specifically, FIG. 5 depicts pairwise similar and dissimilar type interactions of BCC V and FCC Ni.

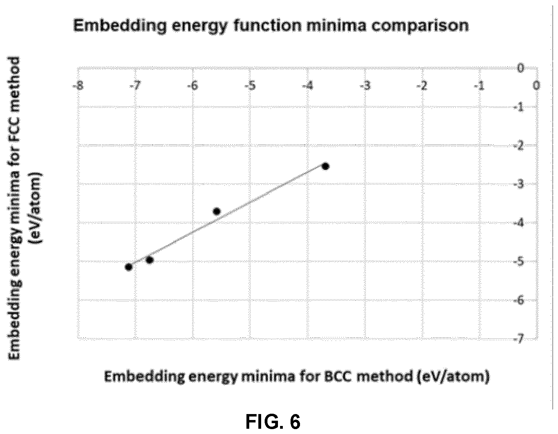

According to the experiments conducted by the present disclosure, it was observed that the Embedding Energy minima value does not require scaling. The Embedding energy minima values from BCC method depends on latest available input parameter values of cohesive energy and vacancy formation energy. However, in FCC models the input cohesive energy was taken from density functional theory (DFT) based calculations. Both the BCC and FCC methodology-based set of values were linearly and proportionately dependent on each other. This gets reflected in the graph of Embedding Energy minima function for both the BCC and FCC MDP methods shown in FIG. 6. FIG. 6, with reference to FIGS. 1 through 5, depicts a graphical representation illustrating a comparison of Embedding energy minima at equilibrium electron density $(\rho_e)$ of elements calculated by BCC and FCC MDP framework, using the system of FIG. 1, in accordance with an embodiment of the present disclosure. More specifically, FIG. 6 shows the validation of applicability of combining the Embedding functions of both BCC and FCC MDP development methods.

Referring to steps of FIG. 2, at step 210 of the present disclosure, the one or more hardware processors 103 identify one or more dissimilar-type pair interaction potential parameters based on the set of scaled parameters and the elemental interaction pair potential function. By combining the BCC and FCC MDP development methods to create the combined BCC-FCC MDP, two binary alloy systems of Ni-V and Pd-Mn were considered for this case study. In the studied alloys Ni and Pd are examples of FCC elements, and V and Mn are examples of BCC elements, respectively. Various compositions of these two binary alloy systems were studied to get the desired structural and thermodynamic properties of the alloy system. Lattice Parameter and Cohesive Energy data were calculated from the model were found to be in good agreement with values obtained from simulations using other MDP from existing literature.

At step 212 of the present disclosure, the one or more hardware processors 103 perform sequencing of the one or more identified dissimilar-type pair interaction potential parameters and one or more similar-type pair interaction potential parameters to obtain a sequence of the pair type interaction potential parameters. For example, consider there are 3 elements A, B and C taken for EAM MDP development. On the final MDP file the electron densities, embedding functions and pairwise interactions are to be tabulated in a particular sequence. For example, at the beginning of the MDP file the calculated Embedding Energy functions of the three elements A, B and C can be tabulated one after another. Next comes the section of atomic electron density part of the MDP and the electron densities of the three elements can be placed in the above sequence. Next for the Pairwise Interaction part of the MDP, both similar element and dissimilar element type interactions need to be tabulated. For the given elements A, B and C in the order, their pairwise interactions come in the sequence of A-A, A-B, B-B, A-C, B-C, C-C and so on, in one example embodiment.

At step 214 of the present disclosure, the one or more hardware processors 103 generate a molecular dynamics potential (MDP) file based on the sequence of the one or more type pair interaction potential parameters. In case study on the Ni-V and Pd-Mn system as considered by the present disclosure, the choice of elements was such that one element naturally belongs to the FCC structure and another belonging to the BCC structure. To predict the properties of the alloys of a mixture between BCC and FCC elements, a merging of the MDP functions from both the BCC and FCC MDP framework is essential. The merging of the MDP functions such as Embedding Energy and atomic electron density is done by the scaling factor derived in earlier analysis. Hence, this model framework holds the potential to generate MDP for any alloy systems comprising a mixture of BCC, FCC, and/or other element types.

Once the MDP file is generated, the hardware processors 104 simulate the MDP file to predict at least one of (i) one or more structural properties, (ii) one or more thermodynamic properties, and (iii) one or more mechanical properties of the multi-element alloy structure. Below Table 3 depicts a comparison of the Cohesive Energy and Lattice Parameters of Ni-V alloy system by the results obtained from the developed EAM MDP of the present disclosure and another modified embedded atom potential (MEAM) taken from the existing literature. Other than pure element composition, for the alloys the atomic percentages of their compositions are also given in the 1st column.

Table 3

| Ni-V system | EAM MDP of the present disclosure | | MEAM based on literature | |
|---|---|---|---|---|
| | Cohesive Energy (eV/atom) | Lattice parameter (Å) | Cohesive Energy (eV/atom) | Lattice parameter (Å) |
| Pure Ni | 4.446 | 3.518 | 4.45 | 3.521 |
| Pure V | 6.493 | 3.037 | 5.3 | 2.999 |
| $Ni_{80}V_{20}$ | 4.885 | 3.535 | 4.746 | 3.608 |
| $Ni_{70}V_{30}$ | 5.07 | 3.632 | 4.8465 | 3.62 |

(continued)

|  | EAM MDP of the present disclosure | | MEAM based on literature | |
|---|---|---|---|---|
| $Ni_{20}V_{80}$ | 4.83 | 3.508 | Phase unstable | - |

Similarly, Pd-Mn alloy systems has been studied by the EAM MDP of the present disclosure and compared with the results obtained from the EAM MDP taken from existing literature. The comparisons of Cohesive Energy and Lattice Parameters of the Pd-Mn system is shown in Table 4. Here the cohesive energies obtained from the EAM MDP of the present disclosure is found to be even better than the MDP form existing literature. It is evident by the largely deviating Cohesive Energy of Mn obtained from the MDP form literature, unlike the close estimate obtained from the EAM MDP of the present disclosure as shown in Table 4.

Table 4

| Mn-Pd system | EAM MDP of the present disclosure | | EAM based from literature | |
|---|---|---|---|---|
| | Cohesive Energy (eV/ atom) | Lattice parameter (Å) | Cohesive Energy (eV/ atom) | Lattice parameter (Å) |
| Pure Pd | 3.907 | 3.89 | 5.2 | 3.92 |
| Pure Mn | 4.614 | 2.848 | 9.056 | 2.78 |
| $Mn_{50}Pd_{50}$ | 4.223 | 2.973 | 5.3927 | 2.998 |
| $Mn_{30}Pd_{70}$ | 3.56 | 3.535 | 4.867 | 3.895 |
| $Mn_{20}Pd_{80}$ | 3.377 | 3.535 | 4.795 | 3.907 |

It is to be understood by a person having ordinary skill in the art or person skilled in the art that examples of the multi-element alloy structures (NiV and/or PdMn), and the one or more input physical parameters corresponding to the above-mentioned alloy structures shall not be construed as limiting the scope of the present disclosure.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method, comprising:

   obtaining, via one or more hardware processors, one or more input physical parameters corresponding to a multi-element alloy structure (202);
   classifying, via the one or more hardware processors, the one or more input physical parameters as one of a first element type, or a second element type to obtain at least one of a first set of elements and a second set of elements respectively (204);
   computing, via the one or more hardware processors, one or more of (i) an embedding energy function, and (ii) an atomic electron density for each input physical parameter of the at least one of the first set of elements and the second set of elements (206);
   scaling, by using a scaling factor, via the one or more hardware processors, the one or more of (i) the embedding energy function, and (ii) the atomic electron density, computed for each input physical parameter of the at least one of the first set of elements and the second set of elements, to obtain a set of scaled parameters and an elemental interaction pair potential function (208);
   identifying, via the one or more hardware processors, one or more dissimilar-type pair interaction potential parameters based on the set of scaled parameters and the elemental interaction pair potential function (210);
   sequencing, via the one or more hardware processors, the one or more identified dissimilar-type pair interaction potential parameters and one or more similar-type pair interaction potential parameters to obtain a sequence of the pair type interaction potential parameters (212); and
   generating, via the one or more hardware processors, a molecular dynamics potential (MDP) file based on the sequence of the one or more type pair interaction potential parameters (214).

2. The processor implemented method of claim 1, further comprising simulating the MDP file to predict at least one of (i) one or more structural properties, (ii) one or more thermodynamic properties, and (iii) one or more mechanical properties of the multi-element alloy structure.

3. The processor implemented method of claim 1, wherein the first set of elements and the second set of elements are distinct from each other.

4. The processor implemented method of claim 1, wherein the first element type and the second element type are one of a body centered cubic (BCC) element type or a face centered cubic (FCC) element type.

5. The processor implemented method of claim 1, wherein the scaling factor is calculated based on a regression analysis of the atomic electron density at embedding energy function minima and equilibrium electron density values.

6. The processor implemented method of claim 1, wherein the order of the sequence of pair type interaction potential parameters is determined based on a sequence of one or more element types comprised in the one or more input physical parameters.

**7.** A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

obtain one or more input physical parameters corresponding to a multi-element alloy structure;
classify the one or more input physical parameters as one of a first element type, or a second element type to obtain at least one of a first set of elements and a second set of elements respectively;
compute one or more of (i) an embedding energy function, and (ii) an atomic electron density for each input physical parameter of the at least one of the first set of elements and the second set of elements;
scale, by using a scaling factor, the one or more of (i) the embedding energy function, and (ii) the atomic electron density, computed for each input physical parameter of the at least one of the first set of elements and the second set of elements, to obtain a set of scaled parameters and an elemental interaction pair potential function;
identify one or more dissimilar-type pair interaction potential parameters based on the set of scaled parameters and the elemental interaction pair potential function;
sequence the one or more identified dissimilar-type pair interaction potential parameters and one or more similar-type pair interaction potential parameters to obtain a sequence of the pair type interaction potential parameters; and
generate a molecular dynamics potential (MDP) file based on the sequence of the one or more type pair interaction potential parameters.

**8.** The system of claim 7, wherein the one or more hardware processors are further configured by the instructions to simulate the MDP file to predict at least one of (i) one or more structural properties, (ii) one or more thermodynamic properties, and (iii) one or more mechanical properties of the multi-element alloy structure.

**9.** The system of claim 7, wherein the first set of elements and the second set of elements are distinct from each other.

**10.** The system of claim 7, wherein the first element type and the second element type are one of a body centered cubic (BCC) element type or a face centered cubic (FCC) element type.

**11.** The system of claim 7, wherein the scaling factor is calculated based on a regression analysis of the atomic electron density at embedding energy function minima and equilibrium electron density values.

**12.** The system of claim 7, wherein the order of the sequence of pair type interaction potential parameters is determined based on a sequence of one or more element types comprised in the one or more input physical parameters.

**13.** One or more non-transitory machine-readable information storage mediums comprising one or more instructions which when executed by one or more hardware processors cause:

obtaining, one or more input physical parameters corresponding to a multi-element alloy structure;
classifying the one or more input physical parameters as one of a first element type, or a second element type to obtain at least one of a first set of elements and a second set of elements respectively;
computing one or more of (i) an embedding energy function, and (ii) an atomic electron density for each input physical parameter of the at least one of the first set of elements and the second set of elements;
scaling, by using a scaling factor, the one or more of (i) the embedding energy function, and (ii) the atomic electron density, computed for each input physical parameter of the at least one of the first set of elements and the second set of elements, to obtain a set of scaled parameters and an elemental interaction pair potential function;
identifying one or more dissimilar-type pair interaction potential parameters based on the set of scaled parameters and the elemental interaction pair potential function;
sequencing the one or more identified dissimilar-type pair interaction potential parameters and one or more similar-type pair interaction potential parameters to obtain a sequence of the pair type interaction potential parameters; and
generating a molecular dynamics potential (MDP) file based on the sequence of the one or more type pair interaction potential parameters .

**14.** The one or more non-transitory machine-readable information storage mediums of claim 13, wherein the one or more instructions which when executed by the one or more hardware processors further cause simulating the MDP file to predict at least one of (i) one or more structural properties, (ii) one or more thermodynamic properties, and (iii) one or more mechanical properties of the multi-element alloy structure.

**15.** The one or more non-transitory machine-readable information storage mediums of claim 13, wherein the first set of elements and the second set of elements are distinct from each other, wherein the first element type and the second element type are one of a body centered cubic (BCC) element type or a face centered cubic (FCC) element type, wherein the scaling factor is calculated based on a regression analysis of the atomic electron density at embedding energy function minima and equilibrium electron density values, and wherein the order of the sequence of pair type interaction potential parameters is determined based on a sequence of one or more element types comprised in the one or more input physical parameters.

SYSTEM
100

MEMORY
102

DATABASE
108

HARDWARE
PROCESSOR(S)
104

INTERFACE(S)
106

**FIG. 1**

obtaining one or more input physical parameters corresponding to a multi-element alloy structure ⌇ 202

classifying the one or more input physical parameters as one of a first element type, or a second element type to obtain at least one of a first set of elements and a second set of elements respectively ⌇ 204

computing one or more of (i) an embedding energy function, and (ii) an atomic electron density for each input physical parameter of the at least one of the first set of elements and the second set of elements ⌇ 206

scaling, by using a scaling factor, the one or more of (i) the embedding energy function, and (ii) the electron density, computed for each input physical parameter of the at least one of the first set of elements and the second set of elements, to obtain a set of scaled parameters and an elemental interaction pair potential function ⌇ 208

identifying one or more dissimilar-type pair interaction potential parameters based on the set of scaled parameters and the elemental interaction pair potential function ⌇ 210

sequencing the one or more identified dissimilar-type pair interaction potential parameters and one or more similar-type pair interaction potential parameters to obtain a sequence of the pair type interaction potential parameters ⌇ 212

generating a molecular dynamics potential (MDP) file based on the sequence of the one or more type pair interaction potential parameters ⌇ 214

**FIG. 2**

FIG. 3

**FIG. 4**

FIG. 5

Embedding energy function minima comparison

FIG. 6

## REFERENCES CITED IN THE DESCRIPTION

**Patent documents cited in the description**

- IN 202121025582